# EUROPEAN PATENT SPECIFICATION

(11) **EP 1 762 379 B1**
(45) Date of publication and mention of the grant of the patent: **13.01.2010**
(21) Application number: 06018777.0
(22) Date of filing: 07.09.2006
(51) Int. Cl.: B32B 27/32, B65D 81/32, A61J 1/00

(54) **Multi-chamber container and process for forming the same**
Mehrkammerbehälter und Verfahren zur Herstellung desselben
Récipient à plusieurs compartiments et procédé de fabrication de celui-ci

(30) Priority: 12.09.2005 JP 2005264383
(43) Date of publication of application: 14.03.2007
(73) Proprietor: Nipro Corporation, Osaka-shi, Osaka 531-8510 (JP)
(72) Inventor: Omori, Kenji, Osaka-shi, Osaka-fu 531-8510 (JP); Kawamura, Shin-ich, Osaka-shi, Osaka-fu 531-8510 (JP); Honda, Minoru, Osaka-shi, Osaka-fu 531-8510 (JP)
(74) Representative: Albrecht, Thomas

(56) References cited:
- EP-A- 1 344 642
- EP-A- 1 602 350
- EP-A- 1 629 974
- EP-A- 1 693 044
- EP-A1- 0 634 270
- EP-A1- 1 106 644
- US-A- 5 865 309

## Description

### Technical Field

This invention relates to a multi-chamber container having a plurality of chambers separated by a partition wall having a peelable seal layer (so called easily peelable seal layer) and, particularly, to a multi-layered container having a plurality of chambers for medical use. More specifically, the invention relates to a multi-chamber container which enables the contents contained in a plurality of chambers to be mixed together by causing the plurality of chambers to communicate with each other upon peeling off of the partition wall having an easily peelable seal layer. Further, this invention relates to a process for forming a multi-chamber container.

### Background Art

Usually, a multi-chamber container having a plurality of small chambers for separately containing liquid agents, powder agents or solid agents has heretofore been formed by using a flexible plastic film. One chamber of a plurality of chambers is partitioned from another adjacent chamber by a partitioning wall that allows communication. The partitioning wall is such that the inner surfaces of the film forming the small chambers are directly melt-adhered together, or are melt-adhered together via an insert film held between the inner surfaces. The partitioning wall easily peels off when an external pressure is applied to the surface of the chambers containing the contents; i.e., interiors of the plurality of chambers communicate to allow the contents contained therein to be mixed together.

As the plastic film (which includes sheet) for forming plural chambers, there has been used a two-layer film including an inner layer of a mixed resin of a straight-chain low-density polyethylene (LLDPE) and a polypropylene (PP) and an outer layer of the straight-chain low-density polyethylene (LLDPE), or a three-layer film including an inner layer of the straight-chain low-density polyethylene (LLDPE) and the polypropylene (PP), an intermediate layer of a mixed resin of the straight-chain low-density polyethylene (LLDPE) and an ethylene/α-olefin elastomer, and an outer layer of the straight-chain low-density polyethylene (LLDPE) (Japanese Patent No. 3060132, Japanese Patent No. 3079403, etc.). In these multi-layered films, the inner layers of the mixed resin of the straight-chain low-density polyethylene (LLDPE) and the polypropylene (PP) are sealed at a melt-adhesion temperature of about 110 to 130°C to form an easily peelable seal layer, and are further sealed at a melt-adhesion temperature of about 170 to 200°C to form a strongly melt-adhered layer.

As the plastic film for forming plural chambers, further, there have been used an aluminum foil laminate (three-layer film of polyester/aluminum foil/polyethylene), a laminated film including a silica-deposited layer (PE/SiO₂/PET/SiO₂/PE) and a polyethylene tubular film (US 5,865,309, US 5,706,937, JP-A-2003-104391). The multi-chamber container is formed of a chamber of the aluminum foil laminate overlaid on the laminated film including the silica-deposited layer, and a chamber of the polyethylene tubular film. The plurality of such chambers are melt-adhered together via a film forming a weakly melt-adhered portion of a polymer blend of a polyethylene and a polypropylene held between the inner surfaces of the polyethylene.

As the weakly melt-adhered portion, a resin having small melt-adhering strength to the polyethylene which is the material forming the innermost layer, for instance, a blend of a polyethylene and a resin with no compatibility thereto is conventionally used.

In the plural chamber container, on the other hand, there arise such problems as a decrease in the medicament content, an increase of the medicament analogues, an increased turbidity of the medicament solution and formation insoluble fine particles which are generated by interaction of the medicine which is an effective component with impurities such as low-molecular components eluted from the linear polyolefin and components eluted from the adhesive layer.

Under such circumstances, the present inventors have already proposed a multi-layered multi-chamber container for medical use which is formed by a multi-layered film including an innermost layer of a cyclic polyolefin or a polymer blend containing the cyclic polyolefin and a second layer of a linear polyolefin adjacent to the above layer (US 2006/0035044) . The multi-layered plural chamber container can be subjected to γ-ray sterilization, decreases the low-molecular components and adhesive components that are eluted out from the multi-layered container to eliminate the above-noted problems.

### Disclosure of the Invention

The present invention provides a seal layer (easily peelable seal layer) used for a multi-chamber container of which at least one chamber chiefly uses a layer of a cyclic polyolefin as an innermost layer, the seal layer being stronglymelt-adhered to the innermost layer of the cyclic polyolefin and being allowed to be peeled off from the layer of the cyclic polyolefin so that the contents in the plurality chambers in the container can be mixed together at the time of use.

The present invention relates to a multi-chamber container having a plurality of chambers formed of a film, wherein one chamber and another chamber are partitioned by a partition wall, characterized in that in at least one chamber, at least an innermost layer of said film is a cyclic polyolefin, and said partition wall comprises an easily peelable seal layer that contains a low-density polyethylene and a propylene/α-olefin copolymer, wherein said easily peelable seal layer is a composition containing a low-density polyethylene and a propylene/α-olefin copolymer at a weight ratio of 80:20 to 20:80.

In this invention, the cyclic polyolefin layer as the innermost layer is amorphous and cannot be weakly melt-adhered to another layer. To impart easily peelable property to a layer, it is conventional to obtain an easily peelable layer by blending the same as the container body with a different material. When the cyclic polyolefin is used for the container body, however, the container body and the sheet made of cyclic polyolefin blended with a material different from the cyclic polyolefin often fail to exhibit an easily peelable property or lose the easily peeling property after being stored for extended periods of time. The present invention uses a sheet made of a blend comprising different materials from the cyclic polyolefin, particularly, a blend of a low-density polyethylene and a propylene/α-olefin copolymer to realize an easily peelable property that enables the contents in the container to be mixed together at the time of use while maintaining the seal strength for extended periods of time.

In the present invention, the multi-chamber container is a container having a plurality of chambers, one chamber being partitioned from another chamber by a partition wall. A specific example may be a container in which a liquid agent container containing a liquid agent and a medicine container liquid-tightly containing a medicament are partitioned from each other by the partition wall having an easily peelable seal layer. For example, the container has a pair of partition walls facing the ends on one of the two chambers, an end of the other chamber being inserted and melt-adhered between the above connecting pieces, and the two chambers being partitioned by the partition walls.

In this invention, at least one of the chambers of the multi-chamber container is formed by a single or multi-layered film in which at least the innermost layer is a cyclic polyolefin. Usually, the chambers separately contain a liquid agent, powdery agent or solid agent. The shape and size of the container may be different depending upon the contents. Other of the chambers of the multi-chamber container may be formed by a single or multi-layered film in which the innermost layer is not a cyclic polyolefin.

In the present invention, the film that constitutes at least one small chamber is a single-layer film or a multi-layered film having at least an innermost layer of the cyclic polyolefin. Preferably, it is a multi-layered film comprising a first layer of the cyclic polyolefin as the innermost layer and a second layer of a linear polyolefin adjacent to the first layer.

The cyclic polyolefin is a thermoplastic saturated norbornene-type polymer which is known (JP-A-4-276253, JP-A-5-317411, JP-A-8-155007, etc.). Specific examples thereof include polymers having structural units represented by the following chemical formula 1 and/or chemical formula 2. 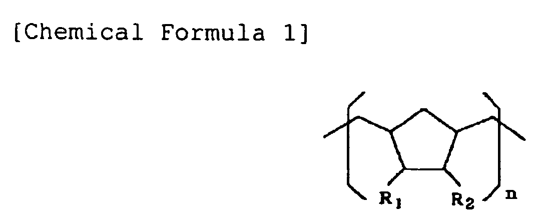 wherein R₁ and R₂ each represents hydrogen atom or a hydrocarbon residue having 1 to 10 carbon atoms and may be the same or different, and R₁ and R₂ together may form a ring, and n is a positive integer.

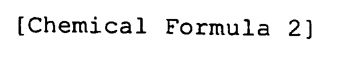

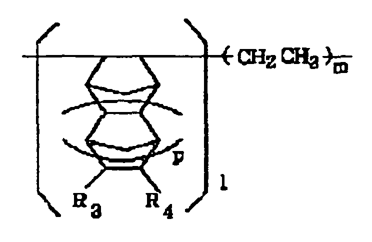

wherein R₃ and R₄ each represents a hydrogen atom or a hydrocarbon residue having 1 to 5 carbon atoms and may be the same or different, and R₃ and R₄ together may form a ring, 1 and m are positive integers, and p is 0 or a positive integer.

The polymer having a structural unit represented by chemical formula 1 is a saturated polymer obtained from a ring-open polymer which is obtained by ring-opening polymerizing a monomer, for example, 2-norbornene and alkyl and/or alkylidene substituted derivatives thereof, such as 5-methyl-2-norbornene, 5,5-dimethyl-2-norbornene, 5-ethyl-2-norbornene, 5-butyl-2-norbornene, 5-ethylidene-2-norbornene; dicyclopentadiene, 2,3-dihydrodicyclopentadiene and derivatives thereof substituted with alkyl such as methyl, ethyl, propyl or butyl; dimethano-octahydronaphthalene and alkyl and/or alkylidene substituted derivatives thereof, such as 6-methyl-1,4:5,8-dimethano-1,4,4a,5,6,7,8,8a-octahydronaph thalene,
6-ethyl-1,4:5,8-dimethano-1,4,4a,5,6,7,8,8a-octahydronapht halene,
6-ethylidene-1,4:5,8-dimethano-1,4,4a,5,6,7,8,8a-octahydro naphthalene; trimer or tetramer of cyclopentadiene, such as 4,9:5,8-dimethano-3a,4,4a-5,8,8a,9,9a-octahydro-1H-benzoin dene,
5,8-methano-3a,4,4a,5,8,8a,9,9a-octahydro-1H-benzoindene, 5,8-methano-1,4,4a,4b,5,8,8a,9b-octahydro-1H-fluorene, and 4, 11:5, 10:6, 9-trimethano-3a, 4, 4a, 5, 5a, 6, 9, 9a, 10, 10a, 11, 11a -dodecahydro-1H-cyclopentaanthracene, and hydrogenating the ring-opened polymer by an ordinarily employed hydrogenation method.

The polymer having a structural unit represented by the chemical formula 2 is a saturated polymer obtained by copolymerizing the above norbornene-type monomer and ethylene according to a known method, and/or a hydrogenated product thereof.

The cyclic polyolefin used in the present invention may be one in which the thermoplastic saturated norbornene-type polymer is a hydrogenated product of a ring-opened polymer of a polar monomer, a copolymer of the above norbornene-type monomer and ethylene, or a hydrogenated product of the above copolymer.

The layer composed of the cyclic polyolefin has a thickness of, usually, 3 to 5000 µm, preferably, 5 to 2000 µm and, when used as a multi-chamber container for medical use, has a thickness of, usually, 10 to 500 µm and, preferably, 20 to 300 µm.

As the second layer composed of the linear polyolefin adjacent to the first layer, there can be exemplified a layer of a linear polyolefin such as low-density polyethylene, straight-chain low-density polyethylene, medium-density polyethylene, high-density polyethylene, polypropylene, or propylene/α-olefin copolymer. The layer of the linear polyolefin has a thickness of, usually, 3 to 5000 µm, preferably, 5 to 2000 µm and, when used as a multi-chamber container for medical use, has a thickness of, usually, 10 to 500 µm and, preferably, 20 to 300 µm.

The film constituting the chambers, as required, further includes a gas-barrier layer and/or a printable layer on the second layer of the linear polyolefin. The printable layer is one on which a medium such as a printing ink can be printed for displaying characters or patterns on the surface of the container. The gas-barrier layer is a layer for preventing the passage of moisture or gases. The printable layer in this invention is, for example, a polyester layer such as polyethylene terephthalate or a polyester layer on which an inorganic matter such as silica is deposited. The gas-barrier layer is, specifically, a metal foil, a metal-deposited layer or an inorganic material-deposited layer. The metal foil may be, for example, an aluminum foil. As the metal-deposited layer, there can be exemplified an alumina-deposited polyester film or a nylon film. The inorganic material-deposited layer may be a silica-deposited polyester film.

In the present invention, the films forming the γ chambers have been adhered together by the easily peelable seal layer. The easily peelable seal layer means a layer that can be easilypeeled of f at the time of preparing a medicine. Usually, the easily peelable seal layer easily peels when an external pressure is applied to the surface of one of chambers containing the contents, enabling the interiors of the plurality of chambers to communicate with each other and the contents in the containers to be mixed together.

The partition wall for partitioning the chambers in the present invention provides a weakly melt-adhered seal portion formed of the above-mentioned easily peeling seal layer and a strongly melt-adhered seal portion.

The weakly melt-adhered seal portion in the present invention is the easily peelable seal layer which is a composition containing the low-density polyethylene and the propylene/α-olefin copolymer.

In the present invention, the low-density polyethylene may be a low-density polyethylene obtained by polymerizing an ethylene monomer in a high-pressure radical polymerization method using such a radical initiator such as an organic peroxide. The low-density polyethylene is a low-density polyethylene having a melt flow rate (MFR) in a range of 0.5 to 8.0 g/10 min. and a density of 0.900 to 0.930. Examplesthereofinclude"Petrothene"manufactured by Toso Co., "Novatec" manufactured by Japan Polyethylene Co., "Excellen" manufactured by Sumitomo Kagaku Co., "UBE Polyethylene" manufactured by Ube-Maruzen Polyethylene Co., and "Mirason" manufactured by Prime Polymer Co. As the α-olefin in the propylene/α-olefin copolymer of the present invention, further, there can be used an α-olefin having 2 or 4 to 8 carbon atoms or, specifically, ethylene, butene, pentene, hexene, heptene, octene or two or more of them. That is, the propylene/α-olefin copolymer of the present invention may specifically be a dicopolymer such as propylene/ethylene copolymer or propylene/butene copolymer, or a tercopolymer such as propylene/ethylene/butene copolymer. The propylene/α-olefin copolymer of the present invention has a melting point in a rangeof, for example, 100°C to 150°C. As the propylene/α-olefin copolymers that have now been placed on the market, there can be exemplified "Novatec PP" and "Wintec" manufactured by Japan Polyethylene Co., "Excellen" manufactured by Sumitomo Kagaku Co., and polypropylenes manufactured by San Alomer Co. and Prime Polymer Co.

It is desired that the mixing ratio by weight of the low-density polyethylene and the propylene/α-olefin copolymer be 80:20 to 20:80, more preferably, 75:25 to 25:75 and, most preferably, 70:30 to 30:70. When the amount of the low-density polyethylene exceeds 80% by weight, the weakly melt-adhered seal that is formed by the film is too strong. When the amount thereof is less than 20% by weight, the weakly-adhered seal strength loses stability.

The partition wall (also called an easily peelable sheet or seal layer) of the invention is prepared from a mixture of a low-density polyethylene and a propylene/α-olefin copolymer in the usual manner. Though there is no particular limitation on the method for forming the mixture, there is preferably used a method for dry-blending resin pellets in advance or a melt-blending method using a biaxial extruder.

In order to improve gas-barrier properties, an easily peelable properties and mechanical properties, the easily peelable sheet of the present invention may be a laminated film having the easily peelable seal layer as at least one surface layer and another resin layer thereon. The number of said easily peelable seal layer and the other resin layers is suitably selected depending upon use. Specifically, other resins are thermoplastic resins such as straight-chain low-density polyethylene resins and cyclic polyolefin-type resins, or a mixture of these resins.

The easily peelable sheet of the present invention can be produced in the form of a sheet by a molding method that is used for general thermoplastic resins, such as T-die molding or inflation molding. Further, the easily peelable laminated-layer sheet can be produced by co-extrusion molding, dry lamination or extrusion coating.

It is desired that the easily peelable sheet of the present invention obtained by the above methods has a thickness of about 5 to about 500 µm and, more preferably, 20 to 400 µm. When the thickness is less than 5 µm, the strength of the film becomes insufficient and it becomes difficult to form an excellent easily peelable seal layer. When the thickness is greater than 500 µm, on the other hand, the flexibility of the film decreases and it becomes difficult to melt-adhere the film.

The easily peelable laminated sheet of the present invention has a thickness of 10 to 500 µm and, preferably, 20 to 400 µm. In the above sheet, the portion occupied by the sheet having the easily peelable property is not smaller than 5% and, preferably, not smaller than 10% of the thickness of the sheet as a whole. When the portion is not larger than 5%, the peeling strength of the easily peelable seal layer formed by the sheet decreases.

In the present invention, strong melt-adhesion means an adhesion formed by heat-melting and adhering the films forming the chambers to liquid-tightly contain the contents therein and which cannot be peeled off by pressure exerted on the external side of the chamber. To strongly melt-adhere the layers of the cyclic polyolefin forming the innermost layer of the present invention, the melt-adhesion is accomplished, for example, at 150 to 200°C for 1 to 3 seconds under a pressure of 0.2 to 0.5 MPa. To maintain the quality, for example, to guarantee the product for three years, it is desired to maintain a T-peel strength of not smaller than 20 N/15 mm after being maintained at 60°C for 7 weeks.

When it is necessary to guarantee the quality of the product sterilized with radioactive rays for 3 years, the strength at 60°C for 7 weeks (= 49 days) is conducted in compliance with Guideline for Industrial Radiation Sterilization of Disposable Medical products (IAEA: TEC DOC-539(1990)) to confirm the effect in short periods of time. T-peel strength for 7 days at 60°C is equivalent to that for 180 days under room temperature.

In the present invention, the weak melt-adhesion is an adhesion such that the partition wall adheres together or the connecting piece adheres to the innermost layers of the films forming the chambers, and can be easily peeled off at the time of preparing a medicine. The partition wall comprising a blend of the low-density polyethylene and the propylene/α-olefin copolymer is weakly melt-adhered together, or the partition wall is weakly melt-adhered to the layer of the cyclic polyolefin, for example, at 130 to 170°C for 0.5 to 2 seconds under a pressure of 0.1 to 0.3 MPa. To maintain the quality of weakly melt-adhered seal strength, for example, it is desired that a T-peel strength is in a range of 0.5 to 3 N/15 mm after being kept at 60°C for 7 weeks.

There is no particular limitation on the method for forming the easily peelable seal layer of the present invention, and there can be used any conventional method for making easily peelable seal layer. Namely, there can be exemplified the following methods.
1) A small piece of film constituting an easily peelable seal layer is strongly melt-adhered onto the innermost layer of cyclic polyolefin of one film, and then, the innermost layer of cyclic polyolefin of another film is weakly melt-adhered onto the above small piece of film.
2) A small piece of film constituting the easily peelable seal layer is inserted between two films having the innermost layers of cyclic polyolefin, and a temperature differential is imparted to metal molds, so that a strong melt-adhesion is accomplished on the side of one film and a weak melt-adhesion is accomplished on the side of the other film.
3) The small piece of film constituting the easily peelable seal layer is weakly melt-adhered onto the innermost layer of cyclic polyolefin of one film, and then, the other film is strongly melt-adhered onto the above small piece of film.

There is no particular limitation on the method for producing the chambers of the multi-chamber container of the present invention, and there can be employed any conventional method concerning with the formation of such chambers. Namely, there can be exemplified the following methods.
1) Two films are overlaid back and front. A film constituting the easily peelable seal layer is held between the two films at a portion slightly above the lowermost end of the films and both the right and left side portions of the two films, except the lower ends thereof, are strongly melt-adhered together so as not to be peeled off. Next, the easily peelable seal layer is weakly melt-adhered to the innermost layers of the films. Thus, the upper sides of the two films form a bag that has an opening upward, and the lower end portions thereof become a pair of connecting piece portions facing back and forth. The connecting piece portions are opened on both the right and left sides and downward. The two films are weakly melt-adhered to the lower ends of the film constituting the easily peelable seal layer to thereby form a partition portion (weakly melt-adhered seal portion), and the two films are strongly melt-adhered on both the right and left sides so as not to be peeled off.
   Thus, there is formed a bag with the upper sides of the two films opening upward and the lower ends thereof forming a pair of connecting pieces facing back and forth. The connecting piece portions are opened on both the right and left sides and downward. The bag is filled with the medicine, and the upper portions of the two films are strongly melt-adhered together so as not to be peeled off to thereby form a chamber (see US 5,865,309, Fig. 1A to 1D, JP-A-2003-104391, Fig. 3).
2) A film constituting an easily peelable seal layer is inserted in an upper end of a tube that is opened at both end, the tube is weakly melt-adhered to the film constituting the easily peelable seal layer to thereby form a partition portion (weakly melt-adhered seal portion), and the front and back wall portions of the tube are strongly melt-adhered so as not to be peeled off on both the right and left sides thereof. Subsequently, a connection port is inserted in the lower end of the tube and is strongly melt-adhered together so as not to be peeled off, and the remaining opening portion at the lower end of the tube is strongly melt-adhered together so as not to be peeled off.
Next, the small chamber is filled with a liquid through the connection port which is, then, closed with a plug to form a second small chamber (see US 5,865,309, Fig. 2A to 2D, JP-A-2003-104391, Fig. 5).

Generally, there is no particular limitation on the method for connecting the small chambers such as the ones of the present invention prepared as described above, and any widely known production method related to making such chambers can be employed.

In the process for forming a multi-chamber container according to the present invention, the two connecting piece portions of the first small chamber are widely opened downward, the upper end of the second small chamber is inserted in between the two connecting piece portions from the lower side, which are, then, heat-melt-adhered together to connect the two small chambers integrally together (see, US 5, 865, 309, Fig. 3 and 4, JP-A-2003-104391, Fig. 6).

The present invention will now be specifically described by way of Examples.

In the Examples, the T-peel strength is measured using a universal tester (Instron) by spreading both ends of two pieces of films over 180° and holding their ends using chucks that are installed to peel. In this state, one end is pulled upward to measure the force applied to peel the melt-adhered portion. The strength of weak seal is set variously depending upon the size of the container or the state in which it is used. In the case of, for example, a 100-mL container for infusion, the strength of the weak seal for the peeling operation may suitably be 0.5 to 3 N/15 mm.

### Examples 1 to 3.

A cyclic polyolefin (trade name: Zeonor manufactured by Zeon Corp.) was extruded at 250°C to make a film A (60 µm). On this film A (100 mm × 155 mm), a small piece of film made of a blend of a low-density polyethylene (density: 0. 922 g/cm³, trade name: Petrothene manufactured by Toso Co.) and a propylene/α-olefin copolymer (Tm 135°C, trade name: Novatec PP manufactured by Japan Polypropylene Co.) (weight ratios of mixture, 70/30, 50/50, 30/70, 60 mm × 30 mm) was placed, and was strongly melt-adhered thereto for 2 seconds using a metal mold heated to a temperature of 180°C. Next, on the above small piece of film, another cyclic polyolefin film B (100 mm x 155 mm) was placed and was weakly melt-adhered thereto for 1.5 seconds using a metal mold heated to a temperature of 150°C to thereby prepare two pieces of cyclic polyolefin films (A and B) to which small film pieces had been melt-adhered.

After being subjected to the γ- ray sterilization, samples were cut into test pieces of a size of (15 mm x 50 mm), and were measured for their T-peel strengths to peel the small pieces of film from the film B using a universal tester (Instron). The T-peel strengths were also measured after the samples were stored at 60°C for one week, three weeks and 7 weeks. The results were as shown in Table 1.

The T-peel strength between the film A and the small pieces of film (just after the preparation) was 15 N/15 mm.

**[Table 1]**

| Example | LDPE/r-PP (weight ratio) | T-peel strength (N/15 mm) | | | |
|---|---|---|---|---|---|
| | | Just after preparation | 60°C×1W | 60°C×3W | 60°C×7W |
| 1 | 70/30 | 2.2 | 1.3 | 1.3 | 0.9 |
| 2 | 50/50 | 2.1 | 1.5 | 1.0 | 1.0 |
| 3 | 30/70 | 0.9 | 0.8 | 0.6 | 0.6 |

### Examples 4 to 6.

A cyclic polyolefin (trade name: Zeonor manufactured by Zeon Corp.) was extruded at 250°C to make a film A (60 µm). On this film A (100 mm x 155 mm), a small piece of film made of a blend of a low-density polyethylene (density: 0.926 g/cm³, trade name: UBE Polyethylene manufactured by Ube-Maruzen Polyethylene Co.) and a propylene/α-olefin copolymer (Tm 135°C, trade name: Novatec PP manufactured by Japan Polypropylene Co.)(weight ratios of mixture, 70/30, 50/50, 30/70, 60 mm x 30 mm) was placed, and was strongly melt-adhered thereto for 2 seconds using a metal mold heated at a temperature of 180°C. Next, on the above small piece of film, another cyclic polyolefin film B (100 mm x 155 mm) was placed and was weakly melt-adhered thereto for 1. 5 seconds using a metal mold heated at a temperature of 155°C to thereby prepare two pieces of cyclic polyolefin films (A and B) to which the small piece of film had been melt-adhered.

After being subjected to the γ- ray sterilization, samples were cut into test pieces of a size (15 mm x 50 mm), and were measured for their T-peel strengths to peel the small piece of film from the film B using a universal tester (Instron). The T-peel strengths were also measured after the samples were preserved at 60°C for one week, three weeks and 7 weeks. The results were as shown in Table 2.

**[Table 2]**

| Example | LDPE/r-PP (weight ratio) | T-peel strength (N/15 mm) | | | |
|---|---|---|---|---|---|
| | | Just after preparation | 60°C×1W | 60°C×3W | 60°C×7W |
| 4 | 70/30 | 1.6 | 1.0 | 1.0 | 0.6 |
| 5 | 50/50 | 1.9 | 1.5 | 0.7 | 0.9 |
| 6 | 30/70 | 0.6 | 0.6 | 0.6 | 0.6 |

### Comparative Examples 1 to 6.

Samples were prepared using small pieces of film as shown in Table 3 instead of using the small piece of film used in Example 1, and by melt-adhering two pieces of cyclic polyolefin films to the same pieces of film in the same manner as in Example 1. Table 3 shows T-peel strengths between the film B and the small piece of film. In Table 3, the abbreviations have the following meanings:
LDPE(a): Low-density polyethylene having a density of 0.922 g/cm³, trade name: Petrothene manufactured by Toso Co.
LDPE(b): Low-density polyethylene having a density of 0.926 g/cm³, trade name: UBE Polyethylene manufactured by
Ube-Maruzen Polyethylene Co.
LLDPE: Straight-chain linear low-density polyethylene
r-PP: Propylene/α-olefin copolymer
h-PP: Polypropylene homopolymer

**[Table 3]**

| Comparative Example | Polymer | Composition (weight ratio) | T-peel strength (N/15 mm) | | | |
|---|---|---|---|---|---|---|
| | | | Just after preparation | 60°C× 1W | 60°C× 3W | 60°C× 7W |
| 1 | LDPE(a) | 100 | 2.6 | 0.3 | 0.2 | N.D. |
| 2 | LDPE(b) | 100 | 1.0 | 0.4 | 0.2 | N.D. |
| 3 | LLDPE/r-PP | 70/30 | 70.1 | N.D. | N.D. | N.D. |
| 4 | LLDPE/r-PP | 30/70 | 33.3 | N.D. | N.D. | N.D. |
| 5 | LDPE(a)/h-PP | 70/30 | 0.2 | N.D. | N.D. | N.D. |
| 6 | LDPE(a)/h-PP | 30/70 | 0.2 | N.D. | N.D. | N.D. |

### Comparative Examples 7 to 9.

Samples were prepared using small pieces of film as shown in Table 4 instead of using the small piece of film used in Example 1, and by melt-adhering two pieces of cyclic polyolefin films to the films in the same manner as in Example 1. Table 4 shows T-peel strengths between the film B and the small pieces of film. In Table 4, the abbreviations have the following meanings:
LDPE(a): Low-density polyethylene having a density of 0.922 g/cm³, trade name: Petrothene manufactured by Toso Co.
COP: Cyclic polyolefin

**[Table 4]**

| Comparative Example | Polymer | Composition (weight ratio) | T-peel strength (N/15 mm) | | | |
|---|---|---|---|---|---|---|
| | | | Just after preparat ion | 60°C× 1W | 60°C× 3W | 60°C× 7W |
| 7 | LDPE/COP | 70/30 | 1.3 | 0.3 | 0.2 | N.D. |
| 8 | LDPE/COP | 80/20 | 1.4 | 0.3 | 0.3 | N.D. |
| 9 | LDPE/COP | 60/40 | 1.6 | 0.4 | 0.3 | N.D. |

As will be obvious from Tables 1 to 4, the small pieces of film using a blend of low-density polyethylene and the propylene/α-olefin copolymer can maintain easily peelable sealing even after being stored at 60°C for 7 weeks.

### Example 7.

A cyclic polyolefin (trade name: Zeonor, manufactured by Zeon Corp.) was extruded together with a medium-density polyethylene (density of 0.938) using a co-extruder at 250°C to prepare a two-layer film (former 30 µm, latter 20 µm). A polyethylene terephthalate film (manufactured by Mitsubishi Kagaku Co. , 12 µm thick) and a medium-density polyethylene film (density of 0.938, 40 µm thick) were successively laminated on the medium-density polyethylene layer of the two-layer film via a polyolefin-type adhesive resin to prepare a four-layer film (front film).

Next, a cyclic polyolefin (trade name: Zeonor manufactured by Zeon Corp.) was extruded together with the medium-density polyethylene (density of 0.938) using the co-extruder at 250°C to prepare a two-layer film (former 30 µm, latter 20 µm). Separately, an aluminum foil (20 µm thick manufactured by Sun Aluminum Co.) and a polyethylene terephthalate film (16 µm thick manufactured by Toyobo Co.) were laminated using a polyurethane-type adhesive (manufactured by Takeda Pharmaceutical Co.) to obtain a two-layer laminated body. On the medium-density polyethylene layer of the above two-layer film, the aluminum foil of the two-layer laminated body was laminated via a polyolefin-type adhesive resin to prepare a four-layer film (rear film).

The above four-layer film (front film) of a square shape and the above four-layer film (rear film) of a square shape were overlaid on one another, a small piece of film made of a blend of the low-density polyethylene and propylene/α-olefin copolymer (weight ratio of mixture, 70/30) was held between the ends thereof, and one surface thereof was strongly melt-adhered and the other surface was weakly melt-adhered at an upper metal mold temperature of 80°C, at a lower metal mold temperature of 140°C and pressure of 0.3 MPa. Both ends of the base end side were strongly melt-adhered together at 170°C and a pressure of 0.3 MPa to form a medicine chamber (bag-like container 140 mm high and 115 mm wide for the medicament). A non-melt-adhered portion without the above film was left on the base end side of the two films.

The medicine chamber in the form of a bag was subjected the γ-ray sterilization, aseptically filled with 1. 0 g of a powdery antibiotic through an end thereof, and the end was strongly melt-adhered together to forma bag-like container.

A small piece of filmmade of a blend of low-density polyethylene and propylene/α-olefin copolymer (weight ratio of mixture, 70/30) was inserted in an upper end of a polyethylene tubular film that is opened at both ends, the tubular film was weakly melt-adhered to the above small film to thereby form a partition portion (weakly melt-adhered seal portion), and the front and back wall portions except the partition portion of the tubular film were strongly melt-adhered so as not to be peeled off on both the right and left sides thereof. Subsequently, a connection port was inserted in the lower end of the tubular film and is strongly melt-adhered together so as not to be peeled off, and the remaining opening portion at the lower end of the tubular film was strongly melt-adhered together so as not to be peeled off to form a second chamber.
The non-melt-adhered portions of the medicine chamber were widely opened downward, the upper end of the second small chamber was inserted in between the two non-melt-adhered portions of the medicine chamber from the lower side, which were, then, heat-melt-adhered together to connect the two small chambers integrally together.

## Claims

1. A multi-chamber container having a plurality of chambers formed of a film, wherein one chamber and another chamber are partitioned by a partition wall, **characterized in that** in at least one chamber, at least an innermost layer of said film is a cyclic polyolefin, and said partition wall comprises an easily peelable seal layer that contains a low-density polyethylene and a propylene/α-olefin copolymer, wherein said easily peelable seal layer is a composition containing a low-density polyethylene and a propylene/α-olefin copolymer at a weight ratio of 80:20 to 20:80.

2. The multi-chamber container according to claim 1, wherein said easily peelable seal layer is an easily peelable single-layer film or an easily peelable a multi-layered film having an easily peelable layer on the surface thereof.

3. The multi-chamber container according to claim 1, wherein said partition wall includes a weakly melt-adhered seal portion comprising said easily peelable seal layer and a strongly melt-adhered seal portion.

4. The multi-chamber container according to claim 1, wherein the film constituting said chambers is a multi-layered film including a first layer of a cyclic polyolefin which is the innermost layer and a second layer of a linear polyolefin adjacent to said layer.

5. The multi-chamber container according to claim 4, wherein the film constituting said plurality of chambers further has a gas-barrier layer and/or a printable layer on the second layer of said linear polyolefin.

6. The multi-chamber container according to claim 5, wherein said gas-barrier layer is a metal foil, a metal-deposited layer or an inorganic material-deposited layer.

7. The multi-chamber container according to claim 5, wherein said printable layer is a polyester layer or a polyester layer having an inorganic material-deposited thin film thereon.

8. The multi-chamber container according to claim 1, wherein said plurality of chambers include one for containing a liquid agent and the one for containing a medicine.

9. The multi-chamber container according to claim 1, wherein said multi-chamber container includes a pair of partition walls facing the ends on one of two chambers, an end of the other chamber being inserted and melt-adhered between the connecting pieces, and the two chambers being partitioned by said partition walls.

10. A process for forming a multi-chamber container according to claim 1, comprising widely opening the two connecting piece portions of the first small chamber downward, inserting the upper end of the second small chamber in between the two connecting piece portions from the lower side which are, then, heat-melt adhered together to connect the two small chambers integrally together.

## Patentansprüche

1. Mehrkammerbehälter mit einer Mehrzahl von Kammern, gebildet aus einer Folie, wobei eine Kammer und eine weitere Kammer durch eine Trennwand abgeteilt sind, **dadurch gekennzeichnet, dass** in mindestens einer Kammer zumindest eine innerste Schicht der genannten Folie ein cyclisches Polyolefin ist, und die genannte Trennwand eine leicht abziehbare Verschlussschicht umfasst, welche ein Polyethylen niedriger Dichte und ein Propylen/α-Olefin-Copolymer enthält, wobei die leicht abziehbare Verschlussschicht eine Zusammensetzung ist, enthaltend ein Polyethylen niedriger Dichte und ein Propylen/α-Olefin-Copolymer mit einem Gewichtsverhältnis von 80:20 bis 20:80.

2. Mehrkammerbehälter gemäß Anspruch 1, wobei die leicht abziehbare Verschlussschicht eine leicht abziehbare Einschichtfolie oder eine leicht abziehbare Mehrschichtfolie mit einer leicht abziehbaren Schicht auf deren Oberfläche ist.

3. Mehrkammerbehälter gemäß Anspruch 1, wobei die Trennwand einen schwach schmelzgeklebten Verschlussbereich, umfassend die leicht abziehbare Verschlussschicht und einen stark schmelzgeklebten Verschlussbereich umfasst.

4. Mehrkammerbehälter gemäß Anspruch 1, wobei die Folie, die die Kammern bildet, eine Mehrschichtfolie ist, umfassend eine erste Schicht aus einem cyclischen Polyolefin, welche die innerste Schicht ist und einer der Schicht benachbarten zweiten Schicht aus einem linearen Polyolefin.

5. Mehrkammerbehälter gemäß Anspruch 4, wobei die die Mehrzahl von Kammern bildende Folie des weiteren eine Gassperrschicht und/oder eine bedruckbare Schicht auf der zweiten Schicht des linearen Polyolefins besitzt.

6. Mehrkammerbehälter gemäß Anspruch 5, wobei die Gassperrschicht eine Metallfolie, eine abgeschiedene Metallschicht oder eine abgeschiedene Schicht aus anorganischem Material ist.

7. Mehrkammerbehälter gemäß Anspruch 5, wobei die bedruckbare Schicht eine Polyesterschicht oder eine Polyesterschicht mit einem darauf abgeschiedenen dünnen Film aus anorganischem Material ist.

8. Mehrkammerbehälter gemäß Anspruch 1, wobei die Mehrzahl von Kammern eine zur Aufnahme eines flüssigen Mittels und eine zur Aufnahme eines Medikaments umfasst.

9. Mehrkammerbehälter gemäß Anspruch 1, wobei der Mehrkammerbehälter ein paar Trennwände umfasst, die den Enden einer der beiden Kammern gegenüberliegen, ein Ende der anderen Kammer eingeführt wird und zwischen den Verbindungsstücken schmelzgeklebt wird, und die beiden Kammern durch die Trennwände abgeteilt werden.

10. Verfahren zur Herstellung eines Mehrkammerbehälters gemäß Anspruch 1, umfassend weites Öffnen der beiden Verbindungsstückbereiche der ersten kleinen Kammer nach unten, Einführen des oberen Endes der zweiten kleinen Kammer zwischen die beiden Verbindungsstückbereiche von der unteren Seite, welche anschließend zusammen schmelzverklebt werden, um die beiden kleinen Kammern gesamtheitlich zusammen zu verbinden.

## Revendications

1. Récipient à plusieurs compartiments ayant une multitude de compartiments formés d'un film, dans lequel un compartiment et un autre compartiment sont séparés par un paroi de séparation, **caractérisé en ce que** dans au moins un compartiment au moins la couche la plus interne du film comprend une polyoléfine cyclique et le paroi de séparation comprend une couche d'étanchéité facilement détachable qui contient un polyéthylène basse densité et un copolymère propylène/α-oléfine, dans lequel la couche d'étanchéité facilement détachable est une composition qui contient un polyéthylène basse densité et un copolymère propylène/α-oléfine avec un rapport pondéral de 80:20 à 20:80.

2. Récipient à plusieurs compartiments selon la revendication 1, dans lequel la couche d'étanchéité facilement détachable est un film monocouche facilement détachable ou un film multicouche facilement détachable ayant une couche facilement détachable sur la surface de celui-ci.

3. Récipient à plusieurs compartiments selon la revendication 1, dans lequel le paroi de séparation comprend une portion d'étanchéité collée à fusion légèrement comprenant la couche d'étanchéité facilement détachable, et une portion d'étanchéité collée à fusion fortement.

4. Récipient à plusieurs compartiments selon la revendication 1, dans lequel le film constituant les compartiments est un film multicouche qui comprend une première couche de polyoléfine cyclique étant la couche la plus interne et une deuxième couche de polyoléfine linéaire adjacente à ladite couche.

5. Récipient à plusieurs compartiments selon la revendication 4, dans lequel le film constituant la multitude de compartiments comprend en outre une couche barrière contre les gaz et/ou une couche imprimable sur la deuxième couche de ladite polyoléfine linéaire.

6. Récipient à plusieurs compartiments selon la revendication 5, dans lequel la couche barrière contre les gaz est une feuille de métal, une couche ayant un dépôt de métal ou une couche ayant un dépôt de matériau inorganique.

7. Récipient à plusieurs compartiments selon la revendication 5, dans lequel la couche imprimable est une couche de polyester ou une couche de polyester disposant du film mince ayant un dépôt de matériau inorganique.

8. Récipient à plusieurs compartiments selon la revendication 1, dans lequel la multitude de compartiments comprend un compartiment destiné à contenir un agent liquide et l'autre compartiment destiné à contenir un médicament.

9. Récipient à plusieurs compartiments selon la revendication 1, dans lequel le récipient à plusieurs compartiments comprend un couple de parois de séparation faisant face aux extrémités à l'un des deux compartiments, une extrémité de l'autre compartiment étant insérée et collée à fusion entre les éléments connectant les deux compartiments étant séparés par lesdits parois de séparation.

10. Procédé pour former un récipient à plusieurs compartiments selon la revendication 1, comprenant les étapes consistantes à ouvrir largement les deux portions d'éléments connectant du premier petit compartiment vers le bas, insérer l'extrémité supérieure du deuxième petit compartiment entre les deux portions d'éléments connectant à partir du côté inférieur, qui sont collés l'un à l'autre par fusion pour lier intégralement les deux petits compartiments.
